# EUROPEAN PATENT APPLICATION

(11) **EP 4 576 118 A1**
(43) Date of publication of application: **25.06.2025**
(21) Application number: 23218125.5
(22) Date of filing: 19.12.2023
(51) Int. Cl.: G16H 50/30, A61B 5/08, A61B 5/00

(54) **COUGH DETECTION AND EVALUATION METHOD AND SYSTEM**

(71) Applicant: SIVA Health AG, 8003 Zürich (CH)
(72) Inventor: MORICE, Alyn Hugh, North Lincolnshire, DN18 5AD (GB); ALGE, Mitja Stefan, 8003 Zürich (CH); DUSCHAU-WICKE, Alexander Herbert Benjamin, 8003 Zürich (CH); KÜTT, Laura, 8908 Hedingen (CH); HÄBERLI KELLER, Michael Andreas, 6043 Adligenswil (CH)
(74) Representative: Maiwald GmbH

(57) **Abstract**

A method for evaluating a coughing activity and a system which allows carrying out the method, as well as a system for evaluating coughing activities, which method and system allow a more reliable evaluation of a coughing activity of a user or a patient.

## Description

### Field of the Invention

The present invention relates to a method for evaluating a coughing activity and a system which allows carrying out the method, as well as a system for evaluating coughing activities, which method and system allow a more reliable evaluation of a coughing activity of a user or a patient.

### Background of the Invention

Coughing is an indication for a disease. Coughing may be acute or may be chronic and may also change over time. The characteristic of coughing may be an indicative for a disease or an intensity of a disease. It may be valuable to evaluate the coughing activity of a patient in order to conclude a disease, so as to treat the disease properly.

For an untrained observer, a coughing activity may be random, and it may be difficult to have an objective measure which may serve for a proper diagnostic. Further, the observation period of a human observer is rather short, as the observation by a human observer lasts only a short time, e.g., only a couple of minutes. For this purpose, sensing and recording devices were used in the prior art, which however did not allow a proper evaluation of the sensed cough activities. Research studies have shown that the averaged frequency of coughing weakly correlates with patient-reported outcomes.

Therefore, there is a need for providing a method and a system for evaluating a coughing activity and to determine a reliable measure for a sensed coughing activity and to characterize cough in a manner that captures personalized insights into the patient's condition.

### Summary of the Invention

The present invention provides a method for evaluating a coughing activity according to the independent claims, whereas further embodiments are incorporated in the dependent claims.

According to an embodiment, there is provided a method for cough evaluation, the method comprising receiving detected cough data representing a cough sequence based on cough events of a patient with corresponding time stamps, and evaluating the cough sequence represented by the cough data, wherein evaluating the cough sequence includes determining a cough free period, wherein a period is considered as cough free period if the period is free of cough events for a at least a predetermined duration.

According to an embodiment, there is provided a method for cough evaluation, the method comprising receiving detected cough data representing a cough sequence based on cough events of a patient with corresponding time stamps, and evaluating the cough sequence represented by the cough data, wherein evaluating the cough sequence includes determining a cough free time as a sum of cough free periods, wherein a period is considered as cough free period for the sum of cough free time if the period is free of cough events for a at least a predetermined duration.

According to an embodiment, there is provided a method for cough evaluation, the method comprising receiving detected cough data representing a cough sequence based on cough events of a patient with corresponding time stamps, and evaluating the cough sequence represented by the cough data, wherein evaluating the cough sequence includes determining a period spent coughing as a period between two subsequent cough free periods, and determining a first cough intensity score as a ratio between a number of cough events during a period spent coughing and a period spent coughing.

A cough or cough event may be considered as an activity of a user or a patient, which activity is a reflex to cough or clear the throat upon a stimulus inside or outside the patient's respiratory tract. Usually, a cough event occurs repeatedly a couple of times with different periods in between where no cough event takes place. The period between two subsequent cough events may be less than a second and may be defined by a period between two subsequent signal peaks obtained from a sensor for sensing cough events. The period between two subsequent cough events may be a break between coughs. The period between two cough events may also last to a couple of minutes and hours before the next cough event occurs upon a returning or upcoming stimulus.

A cough sequence may be considered as a sequence of coughs or cough events. The cough sequence may have a time stamp for some or all cough events. In case cough events are differentiated into different kinds or types of cough events, the cough sequence may have time stamps for only one of few types of cough events or for more than one or all of different types of cough events. Different types of cough events may be used for different evaluations. In particular, different cough events may be, among others, explosive cough sounds, throat clearing sounds, dry cough sounds, wet/productive cough sounds, cough sounds above or below a defined threshold of acoustic energy, different multiplicities of explosive cough sounds in a defined time window (i.e., single, double, triple, ... coughs), cough epochs (i.e., sequences of explosive cough sounds separated by less than 2 seconds).

A time stamp may be allocated to a particular cough event and may be considered as a relative time difference between two cough events or may be an absolute time taken from a clock. A cough sequence may have a number of cough events each having an allocated time stamp so that the relative temporal distribution of the cough events may be given by the cough sequence.

A cough free period may be considered as a period where no cough events occur. A cough free period may be considered for the sum for determining a cough free time if the cough free period is free of cough events for a predetermined duration. This means that a cough free period is considered for the sum only if the period between two subsequent cough events is longer than or at least as long as the predetermined duration. The predetermined duration may be, e.g., 15 minutes. The accumulated length of time greater than a predetermined period, e.g., 15 minutes where no cough events occurred over a monitoring period is then considered for the sum.

A period spent coughing or cough period, respectively, likewise may be considered as a cough period which does not have a period between two subsequent cough events which is longer than the above-described predetermined duration.

A cough free time may be considered as the accumulation of cough free periods over a particular monitoring period. The accumulated sum or length of time greater than a predetermined duration, e.g., 15 minutes where no cough event occurred over a monitoring period, e.g., a 24-hour period may be considered as a cough free time during the 24-hour period.

Likewise, a time spent coughing or cough time may be considered as an accumulation of cough periods or periods spent coughing over a particular monitoring period.

Thus, the method provides an objective measure which may be provided for determining a cough free time and/or a cough intensity when coughing. Considering a cough free period for determining a total cough free time and/or a cough intensity only if the cough free period lasts a predetermined duration may give an objective measure and may allow a comparable and reliable measure, which may allow an instant evaluation of the health conditions, and also an evaluation of the development of a health condition.

According to an embodiment, there is provided a method for cough evaluation, the method comprising receiving detected cough data representing a cough sequence based on cough events of a patient with corresponding time stamps, and evaluating the cough sequence represented by the cough data, wherein evaluating the cough sequence includes determining a cough period as a period between two subsequent cough free periods, wherein a period is considered as cough free period if the period is free of cough events for a at least a predetermined duration.

Thus, the method provides an objective measure which may be provided for determining a cough intensity. Considering a cough free period for determining a cough time in between only if the cough free period lasts a predetermined duration may give an objective measure and may allow a comparable and reliable measure, which may allow an instant evaluation of the health conditions, and also an evaluation of the development of a health condition.

According to an embodiment, the predetermined duration is between 300 seconds and 1800 seconds, in particular between 600 seconds and 1200 seconds, in particular 900 seconds +/-100 seconds.

Thus, a significant and meaningful measure may be provided which allows determining a cough free time. It revealed that the above quantified predetermined duration of cough free time is supported by a patient's perceptions. So called PRO (Patient Reported Outcomes), which are subjective perceptions of a patient relating to the patient's cough suffering revealed correlations between the patient's cough suffering and the above quantified predetermined duration. Relief of cough was assessed by asking 20 patients attending the Hull cough clinic how long a period was before they felt that their bout of coughing had abated. The average was about 15 minutes. This was then retrospectively applied to the data from 24 hour cough counts from a clinical trial. The average cough counts demonstrated no effect of the drug compared to placebo whereas relief of cough (measured as cough free time applying the predetermined duration of 15 minutes) showed a clear improvement in the time not coughing in the drug treatment arm. It also showed a closer correlation with the PROs collected in the study.

According to an embodiment, evaluating the cough sequence comprises determining a first cough free time score, which cough free time score is a ratio between the cough free time and a sensing time during which cough free periods are detected.

A cough score may be considered as a score which quantifies a coughing activity in general.

A cough free time score may be considered as an accumulated sum or length of periods greater than a predetermined duration, e.g., 15 minutes where no cough event occurred over a monitoring period. A cough free time of 6 hours means that, for the predetermined duration of 15 minutes, the sum of all cough free periods longer than 15 minutes times during a monitoring period of 24 hours is in total 6 hours. The cough free time score may be defined as a ratio of the sum of cough free time and the monitoring period where the summed cough free periods were monitored, which ratio for the given example is 6/24, which is 1:4 or 0.25. A higher score usually means longer time spans without cough, which usually means that the disease impact is lower and the health level is higher.

A period spent coughing may be considered as a duration between two subsequent periods not spent coughing as defined above, i.e., as a duration between two subsequent periods where no cough event was identified for at least the predetermined duration. A time spent coughing may be considered as a sum of periods spent coughing.

According to an embodiment, evaluating the cough sequence comprises determining a time spent coughing score, which time spent coughing score may be defined as a ratio between the time spent coughing and a sensing or monitoring duration during which cough periods are detected.

A time spent coughing score may be considered as an accumulated sum or length of periods between cough free periods greater than a predetermined duration, e.g., 15 minutes where no cough event occurred over a monitoring period. A time spent coughing of 12 hours means that, for the predetermined duration of 15 minutes, the sum of all cough periods during a monitoring period of 24 hours is 12 hours. The time spent coughing may be defined as a ratio of the sum of cough periods and the monitoring period, which for the given example is 12/24, which is 1:2 or 0.5. A higher score usually means less time spans without cough, which usually means that the disease impact is higher and the health level is lower.

Thus, a score can be provided which provides a clear and comparable measure for quantifying a disease level.

According to an embodiment, evaluating the cough sequence comprises determining a first cough intensity score as a ratio between a number of cough events during a period spent coughing and that period spent coughing.

According to an embodiment, evaluating the cough sequence comprises determining a second cough intensity score as a ratio between a number of cough events during a time spent coughing and that time spent coughing.

A cough intensity score may be considered as measure how intense the cough periods are. The cough intensity score may be considered as a ratio between a number of cough events and a period or time spent coughing as defined above. In case, e.g., the coughing period or the total coughing time, e.g., during a monitoring period of 24 hours, is 12 hours, and the number of cough events during this in total 12 hours (which is at the same time the number of cough events during the 24-hour monitoring period) is 600, then the cough intensity score would be 600/12 per hour, which is 50 per hour.

According to an embodiment, evaluating the cough sequence comprises determining an average cough intensity score as an average ratio between a number of cough events during a period spent coughing and that period spent coughing.

Thus, an alternative scoring may be provided, in that for each cough period a cough intensity is determined and the intensities than are averaged to provide a measure of cough frequency when coughing.

According to an embodiment, the method further includes receiving patient data, wherein the patient data include data of wearing times representing at least one of the periods when a patient wears a device for collecting cough data and a wearing compliance a patient wears a device for collecting cough data.

A wearing time may be considered as a period, where a patient wears cough data recording device with the sensors. This can be monitored by identifying typical motions indicating that a patient wears the device. A device status may include not only the technical status of the device, like charging status, connectivity status, self-check monitored operating status etc., but also the wearing status of the device.

Wearing compliance may be considered as an intensity of a wearing duration of the cough detection device by a user. A wearing compliance may be considered as having a maximum in case the user wears the cough detection device non-stop. A wearing compliance may be considered as an average wearing time over a predetermined monitoring period.

Thus, more reliable evaluation results may be expected in that the more relevant durations or intervals are stronger considered than less relevant durations or intervals. For the evaluation also the device status and the wearing status may be considered in that only those periods are considered for evaluation where it is detected that the patient wears the device and/or it is detected that the device works in good order. In case the wearing status is not clear, a probability may be determined on how likely it is that the device is worn. According to this probability or a quantum of wearing time during a day, the respective day may be weighted in, e.g., a weekly or monthly aggregation according to wearing time. Likewise, a weighting may take place according to daytime and nighttime. Evaluation may also include a comparison of same daily intervals between two instances.

According to an embodiment, evaluating the cough sequence comprises weighting at least one of periods spent coughing and cough free periods depending on wearing times or wearing compliance.

Thus, those periods can be ignored or at least less weighted where the device is not worn or not properly worn.

According to an embodiment, evaluating the cough sequence comprises determining at least one confidence score for an interval during a cough sequence, wherein the at least one confidence score for an interval during a cough sequence represents a measure in how far the interval during the cough sequence is considered as confident.

A confidence score may be considered as an integral of different factors which may have an impact on the reliability of the evaluation result. The confidence score may include at least one of wearing time, wearing compliance, wearing probability, device status and periods where the patient's symptoms are more typical for a particular disease than in other periods. For the evaluation low scored intervals with a confidence score below a particular threshold may be excluded from consideration, or the intervals may be weighted with the confidence score or an algorithm which relates to the confidence score. Consideration of, e.g., days with an integral confidence score for that day below a particular threshold may be excluded or less weighted for an aggregation, e.g., a weekly or monthly aggregation.

According to an embodiment, an interval during the cough sequence is considered as confident, if at least one the following applies: the interval during the cough sequence falls in a wearing time, the interval during the cough sequence has a detected wearing compliance above a predetermined threshold, the interval during the cough sequence has a patient's self-reported wearing compliance above a predetermined threshold, the confidence level of an interval of a cough sequence is above a predetermined threshold.

Thus, more reliable evaluation results may be expected, as only the confident intervals are considered as relevant or will be stronger weighted than less confident intervals.

According to an embodiment, an interval during the cough sequence is considered as confident, if the confidence level of an interval of a cough sequence is above a predetermined threshold, wherein the confidence level is provided by a neural network detecting a cough sequence, wherein the confidence level in particular corresponds to a confidence level of the neural network.

A confidence level of a neural network may be considered as a probability with which a neural network determines a result.

Thus, a confidence score with a higher complexity considering more factors may be provided. The neural network may be trained with a plurality of labeled data. The higher the variety of the labels, the higher may be the complexity of the confidence score.

According to an embodiment, evaluating the cough sequence comprises weighting intervals of the cough sequence depending on the confidence score.

According to an embodiment, detected cough data are generated based on at least one of an audio signal and an acceleration signal recorded from a patient. In particular, detected cough data may be generated based on either an audio signal alone or an acceleration signal alone or both signals.

Thus, a sensor setup may be used which does not hinder the patient in daily life and which may avoid any invasive placement in the respiratory tract, such as a flow or pressure sensor close to mouth and nose or in the trachea. A recorded audio signal may be analyzed upon typical characteristics for a cough event. These characteristics may be a particular spectra pattern, intensity or amplitude pattern or a combination thereof. Likewise, a motion sensing, in particular an acceleration sensing and recording may be used for identifying characteristic accelerations which are symptomatic for a particular cough event. The audio signal evaluation and the acceleration signal evaluation may be mutually used for plausibility checks. Particularly, the audio signal evaluation and the acceleration signal evaluation may be correlated so that a higher reliability in the cough sequence detection can be achieved.

According to an embodiment, the received patient data further include at least one of habits relating to food intake, habits relating to physical activity, subjective self-reported health conditions and objective health conditions, wherein evaluating the cough sequence comprises determining a correlation between any type of patient data and at least one of the cough free time score and the cough intensity score. The health conditions and other additional information may also come from another data source, e.g., as an input from a health care professional, e.g., a nurse or a doctor.

Thus, also the patient data, in particular self-recorded patient data may be used for checking the plausibility of the evaluation. Further, the patient data may be used for training a neural network, which again may improve the reliability of the confidence score. For recording or input of patient data, a user interface may be used, e.g., a smartphone with a respective application on it. A patient may enter subjective impressions on its health condition. Additionally, health care analyzing functions of the smart phone may be used, as well as other sensorics, which may help analyzing day and night times and other habits of the patient. The cough scores as described above may be correlated with the patient's reported assessment of cough. The score may be calibrated to the respective patient.

According to an embodiment, the method further comprising communicating at least one of the following: receiving input of a health care professional, receiving input of a patient, transmitting output to a health care professional and transmitting output to a health care professional, wherein the output in particular includes at least one of a cough intensity score and a cough free time score, in particular on a daily, a weekly or a monthly basis.

Thus, it is possible to give feedback to the patient and/or a health care professional and to monitor the activities, in particular via the user interface, the smartphone or the dashboard.

According to an embodiment, there is provided a system for carrying out the method as described above, wherein the system is implemented as a cloud-based software.

According to an embodiment, there is provided a system for carrying out the method as described before, wherein the system comprises a receiving section being configured for detecting cough data representing a cough sequence based on cough events of a patient with corresponding time stamps, and an evaluation section being configured to evaluate the cough sequence represented by the cough data; wherein evaluation section is configured for determining a cough free time as a sum of cough free periods, wherein a period is considered for the sum of cough free time if the period is free of cough events for at least a predetermined duration.

According to an embodiment, there is provided a system for carrying out the method as described before, wherein the system comprises a receiving section being configured for detecting cough data representing a cough sequence based on cough events of a patient with corresponding time stamps, and an evaluation section being configured to evaluate the cough sequence represented by the cough data; wherein the evaluation section is configured for determining a cough period as a period between two subsequent cough free periods, wherein a period is considered as cough free period if the period is free of cough events for a at least a predetermined duration.

According to an embodiment, the system is configured for determining a cough free time score, which cough free time score is a ratio between the cough free time and a sensing time during which cough free periods are detected.

According to an embodiment, the system is configured for determining a cough intensity score as a ratio between a number of cough events during a time spent coughing and that time spent coughing.

According to an embodiment, the system is configured for determining an averaged cough intensity score as an average ratio between a number of cough events during a period spent coughing and that period spent coughing.

According to an embodiment, the system is configured for receiving patient data, wherein the patient data include data of wearing times representing at least one of periods when a patient wears a device for collecting cough data and a wearing compliance a patient wears a device for collecting cough data.

According to an embodiment, the system is configured for weighting at least one of periods spent coughing and cough free periods depending on wearing times.

According to an embodiment, the system is configured for determining at least one confidence score for an interval during a cough sequence, wherein the at least one confidence score for an interval during a cough sequence represents a measure in how far the interval during the cough sequence is considered as confident.

According to an embodiment, the system is configured for determining an interval during the cough sequence as confident, if at least one the following applies: the interval during the cough sequence falls in a wearing time, the interval during the cough sequence has a wearing compliance above a predetermined threshold and the confidence level of an interval of a cough sequence is above a predetermined threshold.

According to an embodiment, the system is configured for determining an interval during the cough sequence as confident, if the confidence level of an interval of a cough sequence is above a predetermined threshold, wherein the confidence level is provided by a neural network detecting a cough sequence, wherein the confidence level in particular corresponds to a confidence level of the neural network.

According to an embodiment, the system is configured for weighting intervals of the cough sequence depending on the confidence score.

According to an embodiment, the system is configured for generating detected cough data from at least one of an audio signal and an acceleration signal recorded from a patient. In particular, detected cough data may be generated based on either an audio signal alone or an acceleration signal alone or both signals.

According to an embodiment, the system is configured for receiving patient data including at least one of habits relating to food intake, habits relating to physical activity, subjective self-reported health conditions and objective health conditions, wherein evaluating the cough sequence comprises determining a correlation between any type of patient data and at least one of the cough free time score and the cough intensity score.

According to an embodiment, the system is configured for communicating at least one of the following: receiving input of a health care professional, receiving input of a patient, transmitting output to a health care professional and transmitting output to a health care professional, wherein the output in particular includes at least one of a cough intensity score and a cough free time score, in particular on a daily, a weekly or a monthly basis.

The gist of the invention is to provide a solution that is able to determine the actual cough burden. A cough pattern recognition can provide clinically relevant insights beyond just measuring the frequency of coughing.

It should be noted that the above-described embodiments may also be combined and in a combined form provide a synergetic technical effect and synergetic benefits which go beyond the sum of the single technical effects and benefits.

### Brief Description of the Figures

The invention will be described by way of the following drawings:
- Figure 1:: illustrates a schematic overview of a cough sensing section and its connection to a sensor and a cough detection section;
- Figure 2:: illustrates an exemplary detected cough sequence;
- Figure 3:: illustrates an exemplary structure of a cough evaluation system;
- Figure 4:: illustrates an exemplary embodiment of a cough evaluation;
- Figure 5:: illustrates an exemplary setup of procedural units for a method according to an exemplary embodiment of the invention; and
- Figure 6:: illustrates an exemplary structure of a system according to an embodiment of the invention.

It should be noted that same or similar reference numerals illustrate same or similar components. Along these Figures exemplary embodiments of the invention will be described as follows.

### Detailed Description of Exemplary Embodiments

The invention will be described along exemplary embodiments, which are illustrated in the above references figures and will be described in detail in the following.

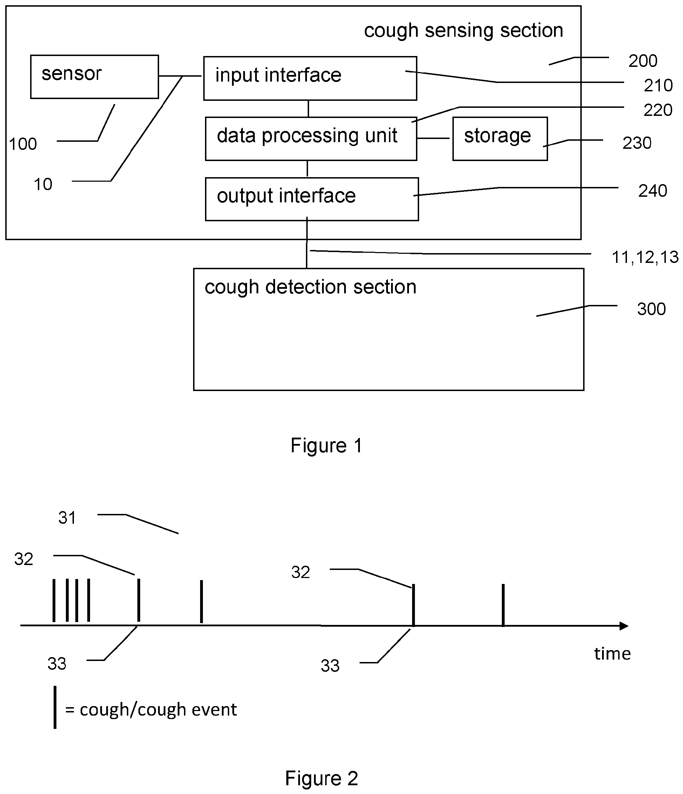

Figure 1 illustrates a schematic overview of a cough sensing section 200 and its connection to a sensor 100 and a cough detection section 300. A sensor 100 may be provided, which may comprise a plurality of different sensor units, which may be capable of measuring different parameters, which may be related to a coughing event. The sensor 100 may be connected to a cough sensing section 200, which is capable of processing sensor data. The cough sensing section 200 may have an input interface 210, a data processing unit 220 and an output interface 240. The cough sensing section 200 may also have a storage 230 for storing data which are useful for the processing of the sensor data 10, e.g., filter algorithms, data processing algorithms etc. The storage 230 may also be used for storing sensor data before outputting the sensor data. This may be required if the connection of the cough sensing section 200 and a further data processing downstream the cough sensing section 200 is interrupted or delayed. The sensor data 10 may be received via the input interface 210, to which the sensor or sensor or sensors 100 may be fixedly or releasably coupled. The sensor data 10, e.g., acoustic data 11, acceleration data 12 etc., then may be provided to the data processing unit 220. After data processing, the data are provided to the output interface 240. The cough sensing section 200 may be connected to a cough detection section 300 via the output interface 240. The cough detection section 300 may be configured for detecting cough events based on the sensor signals 10, 11, 12, 13. The cough detection section 300 may be configured to detect a cough sequence 31 based on the sensor signals 10, 11, 12, 13, as illustrated in Figure 2.

Figure 2 illustrates an exemplary cough sequence 31. The cough sequence 31 may be determined based on the sensor signals 10, 11, 12, 13 sensed by sensors 100. The cough sequence 31 may be composed of a number of cough events 32. The cough events 32 may be identified out of the sensor signals 10, 11, 12, 13. The cough sequence 31 may also include one or more time stamps 33 which may be allocated to a respective cough event 32. The time stamp 33 may be a time difference over a previous time stamp 33 of a previous cough event 32. The time stamp may also be a time difference over an initial time stamp allocated to an initial cough event. The time stamp may also be an absolute time stamp carrying a time taken from a clock. The cough sequence 31 hence is a composition of a number of cough events 32 and their corresponding time stamps 33, which reflect the coughing activity of a patient which is sensed by the sensor 100. The cough detection section 300 may include pattern recognition algorithms for differentiating cough events from other artefacts in the sensor signals 10. The cough detection section 300 may also apply a neural network which can be trained by patient sensor data which are labeled with self-reported patient observation or observations from a health care professional. A neural network which can be used is described, e.g., in WO 2023 041406. The cough detection section 300 may detect different kinds and types of coughs and may also provide a cough sequence 31 with different types of coughs. The cough detection section 300 may also provide different cough sequences 31 relating to different types of cough.

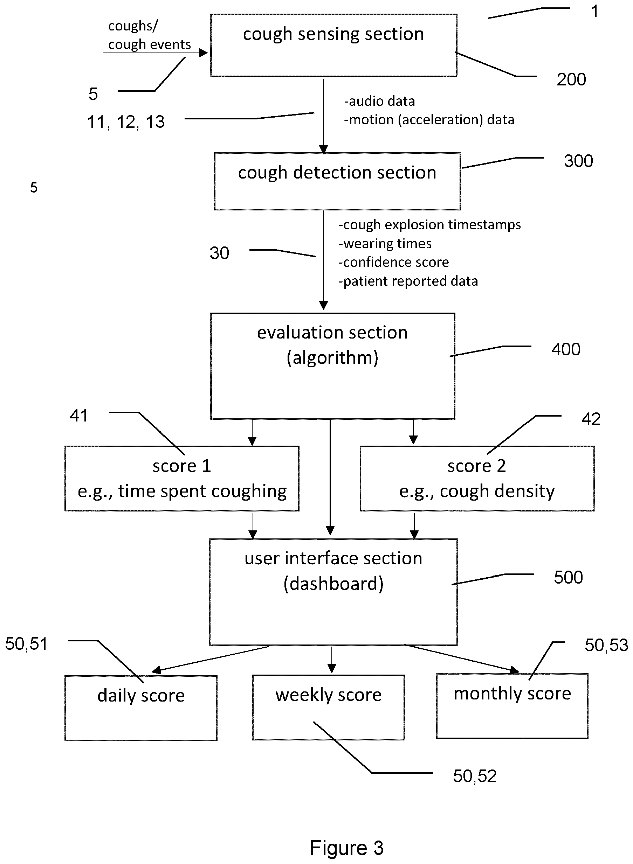

Figure 3 illustrates an exemplary structure of a cough evaluation system. The cough sensing section 200 includes here not shown sensors. The cough sensing section 200 receives coughs or cough events from a patient. The coughs or cough events are then converted into sensing data 10, which may be acoustic sensing data 11 and/or motion sensing data 12, like, e.g., acceleration data. From these data the cough detection section 300 identifies cough events 32 and provides the cough events with respective time stamps 33, so as to provide a cough sequence 31, as described above. The cough sensing section 200 with the sensors 100 may as a cough data recording device be provided as a wearable device which can be worn by a user, in particular a patient. The patient may wear the cough sensing section 200 in form of a clip or an attachment arrangement like a band worn around the neck, so that during a coughing activation the respective signals can be properly sensed. The sensed data 10, 11, 12, 13 then may be transferred to the cough detection section 300. The cough detection section 300 may be provided remotely, e.g., on a server or a cloud. In case the data transfer is delayed or interrupted, the data may be buffered in a storage 230 as described above. The cough detection section 300 then determines a cough sequence as describes above. The cough detection section 300 then provides cough data 30, e.g., the detected cough sequence 31 including the cough events 32 and their respective time stamps 33 to a cough evaluation section 400. The cough detection section 300 may not only provide the cough sequence 31, but also other relevant data, like wearing times or wearing intensities, a confidence score 37 or patient recorded data 38. It should be noted that wearing times or wearing intensities, a confidence score or patient recorded data may also be provided by other sources to the evaluation section 400. The evaluation section 400 may also be provided remote from the cough sensing section 300, e.g., on a server or a cloud. It should be noted that the cough detection section 300 and the evaluation section 400 may be provided on the same server and cloud or different servers and clouds, respectively. The evaluation section 400 may be based on one or more algorithms and may determine scores 41, 42 of a coughing activity. Such scores may be a score relating to time spent coughing or a time not spent coughing or a cough intensity. The respective score 50 may be transferred to a user interface section 500, which may be, e.g., a dashboard 600 which may be inspected by a user, which may be a patient or a health care professional or any other person. The user interface section 500 may provide to the user a score related to a particular time period, e.g., a daily score 51, a weekly score 52, a monthly score 53, or a score for any other time period.

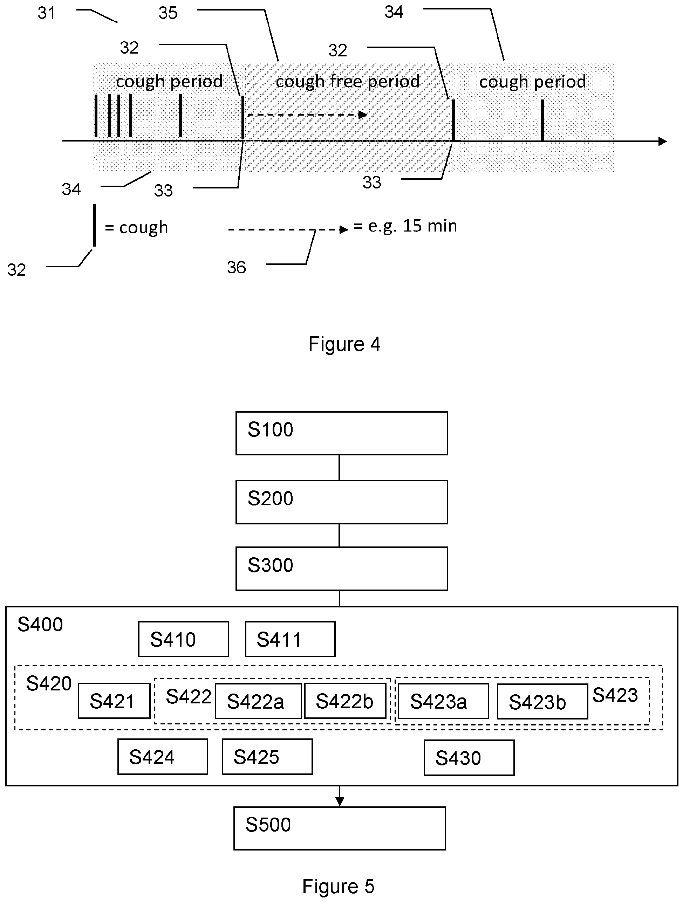

Figure 4 illustrates an exemplary embodiment of a cough evaluation. The sequence 31 of cough events 32 and time stamps 33 corresponds to that of Figure 2. The evaluation section 400 evaluates the chough sequence 31 and its cough events 32 with the corresponding time stamps 33. The evaluation section 400 may determine the periods between subsequent cough events and may determine the temporal distance based on the time stamps 33. As long as the period between two cough events is not longer than a predetermined duration 36, the period is allocated to a respective cough period 34. If the period between two cough events 32 however is longer than a predetermined duration 36, the temporal distance between the two cough events 32 is allocated to a respective cough free period 35, in particular is determined as the cough free period 35. It should be noted that this period may be (and usually is) longer than the predetermined duration 36. The predetermined duration 36 may be between 300 seconds and 1800 seconds, in particular between 600 seconds and 1200 seconds, in particular 900 seconds +/- 100 seconds. Investigations showed that when setting the predetermined duration shorter than the afore-mentioned durations, the impressions of the patients do not correspond to the determined scores. Likewise, investigations showed that when setting the predetermined duration longer than the afore-mentioned durations, the impressions of the patients also do not correspond to the determined scores. 900 seconds +/-100 seconds may be considered as the median. 600 seconds to 1200 seconds may be considered as 75% percentile. 300 seconds to 1800 seconds may be considered as 95% percentile. In order to determine the respective cough time within a monitoring interval, all cough periods 34 within a monitoring interval are added.

Likewise In order to determine the respective cough free time within a monitoring interval, all cough free periods 35 within the monitoring interval are added. The time spent coughing may be considered as a cough score 41, in particular an absolute cough score. The ratio between the cough time and the monitoring interval, e.g., 24 hours, may also be considered as a cough score 41, however, here as a relative cough score. The score may also be defined vice versa, namely focused on the cough free time. In this case the cough free time may be considered as a cough free time score 41, in particular an absolute cough free time score. The ratio between the cough free time and the monitoring interval, e.g., 24 hours, may also be considered as a cough free time score 41, however, here as a relative cough free time score. These afore-described scores express the volume of time which is spent with coughing and time which is spent with not coughing, respectively.

A further score may be provided as a cough intensity score. The cough intensity score is determined by counting the cough events 32 or a particular type of cough events 32 within a cough period 34 and divide the number of cough events 32 by the duration of the cough period 34. This may be considered as a cough density score 42. The cough density score expresses the intensity of a coughing action.

Figure 5 illustrates an exemplary setup of procedural units for a method according to an exemplary embodiment of the invention. Figure 5 includes the method for cough evaluation S400 comprising receiving S410 detected cough data 30 representing a cough sequence 31 based on cough events 32 of a patient with corresponding time stamps 33, and evaluating the cough sequence S420 represented by the cough data 30, wherein evaluating the cough sequence includes determining S421 a cough free time as a sum of cough free periods 35, wherein a period is considered for the sum of cough free time if the period is free of cough events 32 for a at least a predetermined duration 36.

Before the evaluation S400 starts, measurements are to be taken S100 and the coughs or cough events have to be sensed S200. The measurements S100 and sensing of coughing actions S200 then will be undertaken a cough detection S300, where the cough events are identified from the measurements and sensed cough actions. This may take place supported by applying particular algorithms, filters and comparations. This detection may also be supported by applying neural networks or artificial intelligence, which are trained to support the detection procedure.

Evaluating the cough sequence S420 may comprise determining a cough free time score S422 as described above, which cough free time score may be an absolute cough free time score S422a having time as a unit or may be a relative cough free time score S422b as a ratio between the cough free time and a sensing time or interval during which cough free periods 35 are detected. Evaluating the cough sequence S420 may also comprises determining a first cough intensity score S423a as a ratio between a number of cough events 32 during a time spent coughing and a time spent coughing. As an alternative, evaluating the cough sequence S420 may comprise determining a second cough intensity score S423b as an average ratio between a number of cough events 32 during a period spent coughing and a period spent coughing 34. The method may further comprise receiving patient data S430, wherein the patient data 38 include data of wearing times representing at least one of periods when a patient wears a device 1, 200 for collecting cough data and a wearing compliance a patient wears a device 1, 200 for collecting cough data. Received patient data 38 may further include habits relating to food intake, habits relating to physical activity, subjective self-reported health conditions and objective health conditions. Evaluating the cough sequence may comprise determining a correlation between any type of patient data and at least one of the cough free time score and the cough intensity score. Evaluating the cough sequence S420 may comprise weighting S424 at least one of periods spent coughing 34 and cough free periods 35 depending on wearing times. The method may further comprise receiving S411 at least one confidence score 37 for an interval during a cough sequence, wherein the at least one confidence score for an interval during a cough sequence 31 represents a measure in how far the interval during the cough sequence 31 is considered as confident. An interval may be considered as confident if, e.g., it is detected that the device was worn by a patient, that the patient is in a suitable physical condition so that the results are reliable and the like. An interval during the cough sequence 31 is considered as confident, if, e.g., at least one the following applies: the interval during the cough sequence 31 falls in a wearing time, the interval during the cough sequence 31 has a wearing compliance above a predetermined threshold, and the confidence level of an interval of a cough sequence 31 is above a predetermined threshold. The interval during the cough sequence 31 is considered as confident, if, e.g., the confidence level of an interval of a cough sequence 31 is above a predetermined threshold. The confidence level may be provided by a neural network detecting a cough sequence 31, wherein the confidence level in particular corresponds to a confidence level of the neural network. With several neural networks, the confidence level is higher the more neural networks come to the same detection result. Evaluating the cough sequence S420 may comprise weighting S425 intervals of the cough sequence 31 depending on the confidence score.

The method S400 may comprise communicating S440 including, e.g., receiving input of a health care professional, receiving input of a patient, transmitting output to a health care professional and transmitting output to a health care professional, wherein the output in particular includes a cough intensity score and a cough free time score, in particular on a daily, a weekly or a monthly basis. The communication may result from a dashboard action S500, which may include input of data or instructions, output of data or instructions or any combination thereof.

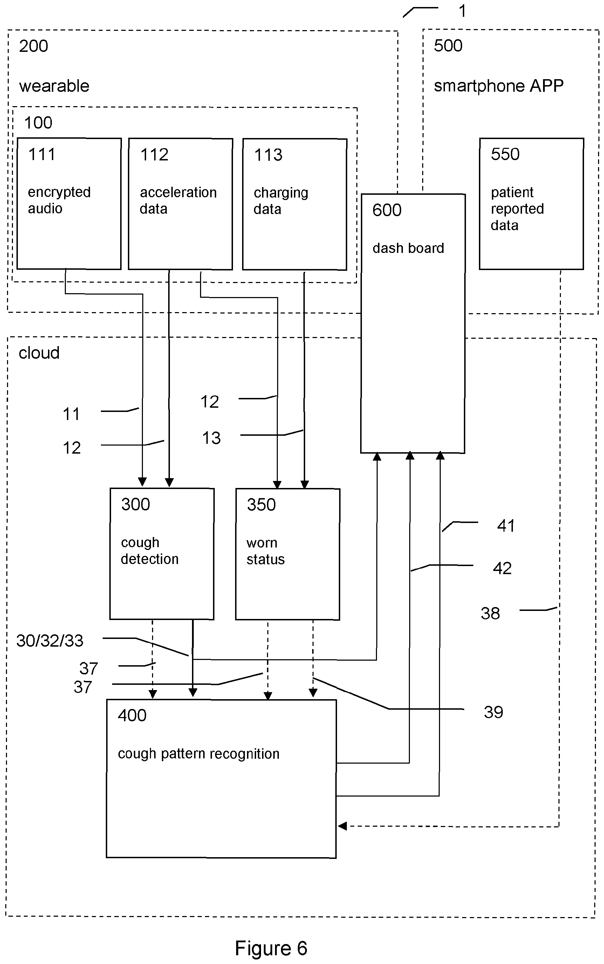

Figure 6 illustrates an exemplary structure a system according to an embodiment of the invention. The system 1 for collecting, processing and evaluating cough data can structurally be considered as a composition of a wearable device 200, a cloud or server based section and a user interface section 500. The user interface section 500 can be realized on a smartphone with an application 550 running on it. The wearable device 200 may have a sensor arrangement 100 with one or more sensors. Figure 6 illustrates an audio sensor 111 for measuring audio signals from the patient wearing the wearable device 200. The audio sensor may output an encrypted audio signal 11 which may be provided for the cough detection in the cough detection section 300. Figure 6 further illustrates a motion sensor 112, in particular an acceleration sensor. This sensor 112 may provide the measured signals 12 for cough detection to the cough detection section 300. The motion signals may also be provided to a to worn status detection section 350, where it may be detected, whether the wearable device 200 is worn or not. Further a charging data senor 113 may be provided which charging data 13 may be provided to the worn status detection section 350. The sensors 111 and 112 detect cough actions, coughs or cough events 5 from the patient wearing the wearable device 200. The measured signals 10, 11, 12, 13 then are handed over to the respective detection section 300, 350. Based on the audio signals 11 and the acceleration signals 12, the cough detection section 300 may determine respective cough data 30, in particular cough sequences 31 with cough events 32 and corresponding time stamps 33 as illustrated in Figure 4. The cough detection section 300 then provides the cough data 30, in particular the cough sequence 31 with the cough events 32 and its time stamps 33 to the cough evaluation section 400, where a cough pattern recognition may take place. The cough detection section 300 may also provide a confidence score 37 to the cough evaluation section 400, which may provide a confidence score based on how reliable the cough detection could be carried out. If the cough detection section 300 for example comes to the conclusion that a cough event 32 is only vague but includes this cough event 32 into the cough sequence31, then the confidence score 37 may be reduced, so that the evaluation section may decide whether or not using these data. In case better data are available from a comparable period, which have a higher confidence score, data with a lower confidence score may be ignored or less weighted. Likewise, the worn status detection section 350 may provide a worn status 39 to the cough evaluation section 400, as well as a confidence score 37, which may result from a vague identification of the worn status.

The cough evaluation section 400 then may evaluate the cough data 30. The cough evaluation section 400 determines from the received data periods spent coughing 34 and cough free periods 35 in the cough sequence 31, as described above with respect to Figure 4. For this purpose, the cough evaluation section 400 applies a predetermined duration 36 as threshold for determining the cough free periods 34 in the cough sequence 31. The predetermined duration 36 may be provided as a set value in the cough evaluation section 400. The predetermined duration 36 may also be modified and/or determined in advance individually for a patient. A health care professional may input a patient specific predetermined duration 36 via the user interface section 500 or a dashboard 600. The predetermined duration 36 may also be adapted for a patient, for example in case the patient has a chronic cough with permanently less than, e.g., 15 minutes between cough events. In this case the predetermined duration 36 may be set for that patient to a reasonable predetermined duration, e.g., 5 minutes, which allows a differentiation of a volatile patient's condition which would not be possible if no cough free periods can be detected due to the longer predetermined duration 36. The cough evaluation section 400 may consider the confidence score 37 received from the cough determining section 300 and/or the confidence score 37 from the worn status determining section 350. The cough evaluation section 400 may then ignore or underweight particular periods 34, 35, in case the detection of cough events are not reliable or confident or a worn status is not reliable or confident. The cough evaluation section 400 may also consider patient data or patient reported data 38, which may input by a patient or a health care professional or any other authorized user via, e.g., the user interface section 500, 550 or dashboard 600. This may be a user input relating to a worn status which is input manually or any other information which may have a relevance for the determination of a cough score 41, 42. The cough evaluation section 400 may determine based on the received data a cough score relating to a time spent coughing as a time spent coughing score/or cough score as described above or a cough score relating to a cough free time as a cough free time score. The cough evaluation section 400 may also determine based on the received data a chough density score 42 as described above. The scores 41, 42 may be output to a user output section or dashboard 600. The dashboard may also be used for user input. The dashboard 600 or in general the user interface 500, 550 may be provided as a smartphone application 550 on a smartphone 500. The dashboard 600 may also be provided on the wearable device 200. The wearable device 200 may be connected (continuously or temporally wired or wireless) to the user interface section or smartphone 500. The dashboard 600 may also be provided as a web-based application and may be provided on the server or cloud. The user, e.g., the patient or health care professional may receive the data of the cough score 41, 42 as an accumulated score 50 on the dashboard 600. The score output via the dashboard 600 may include, e.g., a daily score 51, a weekly score 52 or a monthly score 53. The accumulation may be carried out on the dashboard 600 or on the cough evaluation section, depending on the connectivity, the computational capacities and the data transfer capacity.

Although the cough determining section 300 and the evaluation section 400 are described above as a server or cloud-based solution, the cough determining section 300 and/or the evaluation section 400 may also be provided within the wearable device 200. This may require a larger computational capacity within the wearable device 200. In this case the wearable device 200 may also be connected with a server support for communicating algorithms to be applied or to outsource computational capacities.

### Reference numbers

- 1: system/device for collecting cough data
- 5: coughs / cough events
- 10: signals
- 11: audio signal
- 12: acceleration signal
- 13: charging signal
- 30: cough data
- 31: cough sequence
- 32: cough event
- 33: time stamp / cough time stamp data
- 34: period spent coughing
- 35: cough free period
- 36: predetermined duration as threshold for considering cough free period
- 37: confidence score
- 38: patient data / patient reported data
- 39: worn status
- 41: first score / time spent coughing score / cough score / cough free time score
- 42: second score / shough density score
- 50: score
- 51: daily score
- 52: weekly score
- 53: monthly score
- 100: sensor
- 111: audio sensor
- 112: acceleration sensor
- 113: charging sensor
- 200: cough sensing section, cough data recording device, wearable device
- 210: input interface
- 220: data processing section
- 230: storage
- 240: output interface
- 300: cough detection section
- 350: wearing detection section
- 400: cough evaluation section
- 500: user interface device / smart phone
- 550: user input section / smartphone application
- 600: dashboard
- S100: taking measurement
- S200: sensing chough events
- S300: detecting cough events
- S400: method for cough evaluation
- S410: receiving detected cough data
- S411: receiving confidence score
- S420: evaluating the cough sequence
- S421: determining a cough free time
- S422: determining a cough free time score
- S422a: determining an absolute cough free time score
- S422b: determining a relative cough free time score
- S423: determining a cough intensity score
- S423a: determining a total cough intensity score
- S423b: determining an averaged cough intensity score
- S424: weighting at least one of periods spent coughing
- S425: weighting intervals of cough sequence
- S430: receiving patient data
- S440: communicating input/output/transmission to/from patient/health care professional
- S500: dashboard action

## Claims

1. A method for cough evaluation, the method (S400) comprising:
receiving (S410) detected cough data (30) representing a cough sequence (31) based on cough events (32) of a patient with corresponding time stamps (33);
evaluating the cough sequence (S420) represented by the cough data (30);
wherein evaluating the cough sequence (S420) includes determining a cough free period (35), wherein a period is considered as cough free period (35) if the period is free of cough events (32) for a at least a predetermined duration (36).

2. The method according to claim 1, wherein the predetermined duration (36) is between 300 seconds and 1800 seconds, in particular between 600 seconds and 1200 seconds, in particular 900 seconds +/- 100 seconds.

3. The method according to any one of claims 1 and 2, wherein evaluating the cough sequence includes determining (S421) a cough free time as a sum of cough free periods (35).

4. The method according to claim 3, wherein evaluating the cough sequence (S420) comprises determining a cough free time score (S422), which cough free time score is a ratio between the cough free time and a sensing interval during which cough free periods (35) are detected.

5. The method according to any one of claims 1 to 4, wherein evaluating the cough sequence (31) includes determining a period spent coughing (34) as a period between two subsequent cough free periods (35), and determining a first cough intensity score (S423a) as a ratio between a number of cough events (32) during a period spent coughing (34) and a period spent coughing (34).

6. The method according to any one of claims 3 to 5, wherein evaluating the cough sequence (S420) comprises determining a second cough intensity score (S423a) as a ratio between a number of cough events (32) during a time spent coughing and a time spent coughing, wherein a time spent coughing is a total time minus the cough free time.

7. The method according to any one of claims 1 to 6, wherein evaluating the cough sequence (S420) comprises determining an averaged cough intensity score (S423b) as an average ratio between a number of cough events (32) during a period spent coughing and a period spent coughing (34).

8. The method according to any one of claims 1 to 7, further receiving patient data (S430), wherein the patient data (38) include data of wearing intervals representing at least one of the periods when a patient wears a device for collecting cough data, a wearing compliance a patient wears a device for collecting cough data, and a wearing probability, wherein evaluating the cough sequence (S420) in particular comprises weighting (S424) at least one of periods spent coughing (34) and cough free periods (35) depending on wearing times, the wearing compliance or the wearing probability.

9. The method according to any one of claims 1 to 8, wherein the method further comprises receiving (S411) at least one confidence score (37) for an interval during a cough sequence (31), wherein the at least one confidence score (37) for an interval during a cough sequence (31) represents a measure in how far the interval during the cough sequence (31) is considered as confident.

10. The method according to claim 9, wherein an interval during the cough sequence (31) is considered as confident, if at least one the following applies: the interval during the cough sequence (31) falls in a wearing time, the interval during the cough sequence (31) has a wearing compliance above a predetermined threshold, the confidence level of an interval of a cough sequence (31) is above a predetermined threshold.

11. The method according to claim 9, wherein an interval during the cough sequence (31) is considered as confident, if the confidence level of an interval of a cough sequence (31) is above a predetermined threshold, wherein the confidence level is provided by a neural network detecting a cough sequence (31), wherein the confidence level in particular corresponds to a confidence level of the neural network.

12. The method according to any one of claims 9 to 11, wherein evaluating the cough sequence (S420) comprises weighting (S425) intervals of the cough sequence (31) depending on the confidence score.

13. The method according to any one of claims 1 to 12, wherein detected cough data are generated from at least one of an audio signal (11) and an acceleration signal (12) recorded from a patient.

14. The method according to any one of claims 8 to 13, wherein the received patient data (38) further includes at least one of habits relating to food intake, habits relating to physical activity, subjective self-reported health conditions and objective health conditions, wherein evaluating the cough sequence comprises determining a correlation between any type of patient data and at least one of the cough free time score and the cough intensity score.

15. The method according to any one of claims 1 to 14, further comprising communicating (S440) at least one of the following: receiving input of a health care professional, receiving input of a patient, transmitting output to a health care professional and transmitting output to a health care professional, wherein the output in particular includes at least one of a cough intensity score and a cough free time score, in particular on a daily, a weekly or a monthly basis.
